# EUROPÄISCHE PATENTSCHRIFT

(11) **EP 3 218 152 B1**
(45) Veröffentlichungstag und Bekanntmachung des Hinweises auf die Patenterteilung: **17.05.2023**
(21) Anmeldenummer: 15790463.2
(22) Anmeldetag: 29.10.2015
(51) Int. Cl.: B25J 19/02, B25J 19/04, B25J 9/16

(54) **SYSTEM MIT EINEM MEDIZINISCHEN INSTRUMENT UND EIN AUFNAHMEMITTEL**
SYSTEM WITH A MEDICAL INSTRUMENT AND RECORDING MEANS
SYSTÈME AVEC UN INSTRUMENT MÉDICAL ET UN MOYEN D'ENREGISTREMENT

(30) Priorität: 13.11.2014 DE 102014016843
(43) Veröffentlichungstag der Anmeldung: 20.09.2017
(73) Patentinhaber: KUKA Deutschland GmbH, 86165 Augsburg (DE)
(72) Erfinder: KOGAN, Yevgen, 86152 Augsburg (DE)
(74) Vertreter: Schlotter, Alexander Carolus Paul
(86) Internationale Anmeldenummer: PCT/EP2015/002170
(87) Internationale Veröffentlichungsnummer: WO 2016/074770

(56) Entgegenhaltungen:
- DE-A1-102010 029 275
- DE-A1-102013 109 677
- US-A1- 2013 331 644

## Beschreibung

Die vorliegende Erfindung betrifft ein medizinisches System, das eine Manipulatoranordnung, ein medizinisches Instrument und ein Aufnahmemittel zum Erstellen von Bildern aufweist, wobei das Aufnahmemittel und/oder das Instrument durch die Manipulatoranordnung geführt ist, sowie ein Computerprogrammprodukt zur Durchführung eines Verfahrens zum automatischen Vorgeben einer Soll-Bewegung der Manipulatoranordnung.

Aus der WO 2012/078989 A1 ist ein System mit einem Instrumenten-Roboter, der ein mikroinvasives medizinisches Instrument führt, und einem Kamera-Roboter bekannt, der eine mikroinvasive Kamera führt, welche Bilder eines Operationsgebietes des Instrumentes erstellt.

Die WO 2012/078989 A1 schlägt drei Verfahren zum Vorgeben einer Soll-Bewegung des Kamera-Roboters vor: in einem sogenannten stationären Modus verharrt der Kamera-Roboter stets in einer Position. In einem Folge-Modus wird die Soll-Bewegung des Kamera-Roboters so vorgegeben, dass sich das Instrument bzw. dessen Spitze stets im Zentrum des Blickfeldes der Kamera bzw. des erstellten Bildes des Operationsgebietes befindet. In einem Zoom-Modus wird die Kamera um fest vorgegebene Distanzen von 2 cm entlang einer Verbindungsachse verschoben, sobald ein Blickwinkel zur Erfassung der Spitze des Instruments zu groß oder klein wird.

Die DE 10 2010 029 275 A1 offenbart ein Verfahren zum Bewegen eines Instrumentenarms eines Laparoskopieroboters in eine vorgebbare Relativlage relativ zu einem in einem Patienten platzierten Trokar, bei dem am Trokar ein von außerhalb des Patienten ortbarer Ortsmarker angebracht, die Ortsposition des Trokars anhand des Ortsmarkes erfasst, die Soll-Position des Instrumentenarms aus der Ortsposition und der Relativlage ermittelt, die Ist-Position des Instrumentenarms erfasst und der Instrumentenarm anhand der Ist-Position, der Soll-Position und eines Fehlerminimierungsverfahrens in die Soll-Position bewegt wird, in der er koaxial zur Mittellängsachse des Trokars ist.

Aufgabe der vorliegenden Erfindung ist es, den Betrieb eines gattungsgemäßen medizinischen Systems zu verbessern. Diese Aufgabe wird durch ein medizinisches System mit den Merkmalen des Anspruchs 1 gelöst. Anspruch 8 stellt ein Computerprogrammprodukt zur Durchführung eines hier beschriebenen Verfahrens unter Schutz. Die Unteransprüche betreffen vorteilhafte Weiterbildungen.

Nach einem Aspekt der vorliegenden Erfindung weist ein medizinisches System eine Manipulatoranordnung, ein medizinisches Instrument, ein Aufnahmemittel zum Erstellen von Bildern und ein Steuermittel auf, wobei das Aufnahmemittel, in einer Ausführung das Aufnahmemittel und das Instrument, durch die Manipulatoranordnung geführt ist bzw. wird.

Die Manipulatoranordnung weist in einer Ausführung einen oder mehrere Manipulatoren, insbesondere Roboter, auf. Einer oder mehrere dieser Manipulatoren weisen in einer Ausführung jeweils ein oder mehrere, insbesondere durch das Steuermittel aktuierbare, insbesondere aktuierte, Dreh- und/oder Lineargelenke auf, insbesondere jeweils wenigstens sechs oder mehr Gelenke. In einer Ausführung sind ein oder mehrere Manipulatoren, insbesondere deren Basen, stationär und/oder ein oder mehrere Manipulatoren, insbesondere deren Basen, mobil.

Das medizinische Instrument ist in einer Ausführung zum minimalinvasiven Einsatz ausgebildet. In einer Ausführung weist es einen Instrumentenschaft auf, der, insbesondere durch eine künstliche oder natürliche Körperöffnung, in einen Patienten eingeführt oder dazu vorgesehen bzw. eingerichtet ist, und/oder dessen eingeführter bzw. zum Einführen ausgebildeter Bereich einen maximalen Umfang von höchstens 20 cm, insbesondere höchstens 5 cm aufweist. In einer Ausführung weist das Instrument einen, insbesondere relativ zu dem Instrumentenschaft aktuiert bewegbaren, Endeffektor, insbesondere zum Ein- und/oder Ausführen von Flüssigkeiten und/oder Gasen und/oder zum mechanischen Interagieren, insbesondere Schneiden, Klemmen oder dergleichen auf. In einer Ausführung ist das Instrument, insbesondere sein Instrumentenschaft, mit wenigstens einem Manipulator der Manipulatoranordnung koppelbar, insbesondere, vorzugsweise lösbar, verbunden, und so mittels der Manipulatoranordnung bewegbar bzw. durch diese geführt.

Das Aufnahmemittel ist in einer Ausführung zum minimalinvasiven Einsatz ausgebildet. In einer Ausführung weist es einen Schaft auf, der, insbesondere durch eine künstliche oder natürliche Körperöffnung, in einen Patienten eingeführt oder dazu vorgesehen bzw. eingerichtet ist, und/oder dessen eingeführter bzw. zum Einführen ausgebildeter Bereich einen maximalen Umfang von höchstens 20 cm, insbesondere höchstens 5 cm aufweist. In einer Ausführung weist das Aufnahmemittel einen, insbesondere relativ zu dem Schaft aktuiert bewegbaren oder ortsfesten, Endeffektor auf, der, insbesondere intra- oder extrakorporal, Ultraschall und/oder, insbesondere sichtbare oder unsichtbare, elektromagnetische Strahlung aussendet und/oder empfängt oder dazu vorgesehen bzw. ausgebildet ist. In einer Ausführung ist das Aufnahmemittel, insbesondere sein Schaft, mit wenigstens einem Manipulator der Manipulatoranordnung koppelbar, insbesondere, vorzugsweise lösbar, verbunden, und so mittels der Manipulatoranordnung bewegbar bzw. durch diese geführt. In einer Ausführung verarbeitet das Aufnahmemittel empfangenen Ultraschall und/oder empfangene sichtbare oder unsichtbare elektromagnetische Strahlung weiter, insbesondere, um Bilder, insbesondere von wenigstens einem Teil des Instruments, insbesondere dessen Endeffektor, und/oder einer Umgebung hiervon, zu erstellen, oder ist dazu vorgesehen bzw. ausgebildet. Das Aufnahmemittel kann insbesondere eine Leuchtquelle und/oder, insbesondere elektronische, Kamera aufweisen.

Nach einem Aspekt der vorliegenden Erfindung weist ein Verfahren zum automatischen Vorgeben, insbesondere Bestimmen bzw. Ermitteln, einer Soll-Bewegung der Manipulatoranordnung folgende Schritte auf, die insbesondere durch das Steuermittel durchgeführt werden können:
- Ermitteln einer Soll-Transformation zwischen einer aufnahmemittelfesten Referenz und einer instrumentenfesten Referenz;
- Überwachen einer Abweichung zwischen der Soll-Transformation und einer aktuellen Transformation zwischen der aufnahmemittelfesten Referenz und der instrumentenfesten Referenz; und
- Bestimmen einer Reset-Bewegung der Manipulatoranordnung zur Rückführung der aktuellen Transformation auf die Soll-Transformation, wenn die Abweichung eine, insbesondere variabel, vorgegebene Bedingung erfüllt.

Die Verweise auf therapeutische oder chirurgische Behandlungsverfahren in dieser Beschreibung sind als Verweis auf Computerprogrammprodukte oder medizinische Systeme zu verstehen. Das Steuermittel setzt in einer Ausführung diese vorgegebene Soll- bzw. Reset-Bewegung durch entsprechende Aktuierung von Gelenken der Manipulatoranordnung um oder ist hierzu eingerichtet.

Hierdurch kann das Aufnahmemittel vorteilhaft Bilder von dem Instrument und dessen Operationsgebiet erstellen: Solange die Abweichung zwischen der Soll- und der aktuellen Transformation die vorgegebene Bedingung (noch) nicht erfüllt, wird keine Reset-Bewegung der Manipulatoranordnung bestimmt. Entsprechend kann das Aufnahmemittel in einer Ausführung dann vorteilhaft "umgebungsfeste" Bilder erstellen, was insbesondere eine Steuerung des Instruments durch einen Operateur auf Basis der Bilder erleichtern kann. Ist hingegen die vorgegebene Bedingung erfüllt, wird eine Reset-Bewegung bestimmt, die die aktuelle Transformation auf die Soll-Transformation zurückführt, so dass das Aufnahmemittel nach Ausführen der Reset-Bewegung vorteilhaft Bilder erstellen kann, deren Perspektive bezüglich des Instruments einer Perspektive vor der Reset-Bewegung entspricht, was wiederum insbesondere die Steuerung des Instruments durch den Operateur auf Basis der Bilder erleichtern kann.

Im Gegensatz hierzu "bewegt" sich im Folge-Modus der WO 2012/078989 A1 in den Bildern scheinbar eine Umgebung unter einem ortsfesten Instrument, was die Steuerung des Instruments durch den Operateur auf Basis der Bilder erschwert. Im Zoom-Modus der WO 2012/078989 A1 wird hingegen die Kamera um fest vorgegebene Distanzen entlang der Verbindungsachse verschoben, so dass sich die Perspektive der Bilder in ungünstiger Weise ändert, was ebenfalls die Steuerung des Instruments durch den Operateur auf Basis der Bilder erschwert.

In einer Ausführung ist die aufnahmemittelfeste Referenz (orts)fest bezüglich eines Bauteils des Aufnahmemittels, insbesondere seines Schaftes oder Endeffektors. In einer anderen Ausführung ist die aufnahmemittelfeste Referenz (orts)fest bezüglich eines von dem Aufnahmemittel erstellten Bildes bzw. ortsfest in einem solchen Bild. Beides wird vorliegend zur kompakteren Darstellung verallgemeinernd als aufnahmemittelfeste Referenz bezeichnet. Die aufnahmemittelfeste Referenz kann insbesondere ein Punkt oder ein ein- oder mehrdimensionales Koordinatensystem aufweisen, insbesondere sein.

In einer Ausführung ist die instrumentenfesten Referenz (orts)fest bezüglich eines Bauteils des medizinischen Instruments, insbesondere seines Instrumentenschaftes oder Endeffektors. In einer anderen Ausführung ist die instrumentenfesten Referenz (orts)fest bezüglich eines Abbildes des Instruments in einem von dem Aufnahmemittel erstellten Bild. Beides wird vorliegend zur kompakteren Darstellung verallgemeinernd als instrumentenfeste Referenz bezeichnet. Die instrumentenfeste Referenz kann insbesondere ein Punkt oder ein ein- oder mehrdimensionales Koordinatensystem aufweisen, insbesondere sein.

Eine Transformation ist in einer Ausführung eine Zuordnung zwischen einer aufnahmemittelfesten Referenz und einer instrumentenfesten Referenz, insbesondere eine Abbildung bzw. Abbildungsvorschrift, die die eine Referenz auf die andere Referenz abbildet, insbesondere überführt. Sind in einer einfachen Ausführung die Referenzen ortsfeste Punkte ***x***, ***y*** an Instrument und Aufnahmemittel bzw. (Ab)Bild, so kann der Verbindungsvektor (***x***-***y***) eine solche Transformation sein. Sind die Referenzen in einer Ausführung dreidimensionale Koordinatensysteme ***A***, ***B***, so kann die Abbildung ***T***_{AB}, die den im Koordinatensystem ***B*** beschriebenen Vektor _{B}***x*** in den im Koordinatensystem ***A*** beschriebenen Vektor _{A}***x*** transformiert, eine solche Transformation sein. Eine solche Transformation kann beispielsweise durch den Verbindungsvektor der Ursprünge der beiden Koordinatensysteme ***A***, ***B*** und die Drehmatrix ***Rot***_{AB} zwischen den beiden Koordinatensystemen, die Denavit-Hartenberg-Matrix oder dergleichen beschrieben bzw. definiert sein bzw. werden. Ergänzend wird diesbezüglich auch auf die WO 2012/078989 A1 Bezug genommen und deren Inhalt in die vorliegende Offenbarung einbezogen.

Eine Transformation kann somit insbesondere eine relative Position und/oder Orientierung eines Bauteils des Aufnahmemittels und eines Bauteils des medizinischen Instruments bzw. bauteilfester Referenzen beschreiben bzw. durch diese definiert sein. Gleichermaßen kann eine Transformation insbesondere eine relative Position und/oder Orientierung eines von dem Aufnahmemittel erstellten Bildes und eines Abbildes des medizinischen Instruments in diesem Bild bzw. (ab)bildfester Referenzen beschreiben bzw. durch diese definiert sein.

In einer Ausführung wird die Reset-Bewegung auf Basis der Abweichung bei Erfüllung der Bedingung und/oder unabhängig von einer Relativbewegung zwischen dem Instrument und dem Aufnahmemittel vorgegeben, die nach Erfüllung der Bedingung erfolgt.

Hierdurch beeinflusst in einer Ausführung eine (weitere) Relativbewegung zwischen dem Instrument und dem Aufnahmemittel, die nach Erfüllung der Bedingung erfolgt, die Reset-Bewegung nicht, die auf Basis der Abweichung bei Erfüllung der Bedingung vorgegeben wird. Mit anderen Worten kann ein Aufnahmebereich des Aufnahmemittels wieder auf seine ursprüngliche Position und/oder Orientierung relativ zu dem Instrument (zu)rückgeführt werden, auch wenn sich Instrument und Aufnahmemittel in der Zwischenzeit relativ zueinander weiter bewegen. Zusätzlich oder alternativ kann so eine vorteilhafte, beispielsweise optisch möglichst wenig irritierende, Reset-Bewegung unabhängig von einer zwischenzeitlichen weiteren Relativbewegung zwischen dem Instrument und dem Aufnahmemittel vorgegeben werden. Dies kann insbesondere die Steuerung des Instruments durch den Operateur auf Basis der Bilder erleichtern.

Die Reset-Bewegung zur Rückführung der aktuellen Transformation auf die Soll-Transformation wird in einer Ausführung auf Basis der Abweichung, insbesondere der Abweichung bei Erfüllung der Bedingung, bestimmt, insbesondere derart, dass diese Abweichung sich verringert, vorzugsweise minimal wird, insbesondere derart, dass die Abweichung sich, wenigstens im Wesentlichen, auf Null verringert. Entsprechend ist in einer Ausführung die Abweichung zwischen der Soll- und der rückgeführten aktuellen Transformation, wenigstens im Wesentlichen, gleich Null.

In einer Ausführung wird die Reset-Bewegung mit einer, insbesondere maximalen oder konstanten, Geschwindigkeit vorgegeben, die wenigstens das Doppelte oder höchstens die Hälfte einer, insbesondere aktuellen, maximalen, minimalen oder mittleren, Geschwindigkeit einer Relativbewegung zwischen dem Instrument und dem Aufnahmemittel beträgt. In einer Weiterbildung ist die Geschwindigkeit der Reset-Bewegung variabel einstell- bzw. vorgebbar, insbesondere kann eine automatische und/oder manuelle Auswahl zwischen einem ersten Modus, in dem die Reset-Bewegung mit einer Geschwindigkeit vorgegeben wird, die wenigstens das Doppelte der Geschwindigkeit der Relativbewegung zwischen dem Instrument und dem Aufnahmemittel beträgt, und einem zweiten Modus vorgesehen sein, in dem die Reset-Bewegung mit einer Geschwindigkeit vorgegeben wird, die höchstens die Hälfte der Geschwindigkeit der Relativbewegung zwischen dem Instrument und dem Aufnahmemittel beträgt

Durch die, insbesondere wahlweise, Vorgabe einer Geschwindigkeit, die wenigstens das Doppelte der maximalen Geschwindigkeit der Relativbewegung zwischen dem Instrument und dem Aufnahmemittel beträgt, kann die Reset-Bewegung sehr rasch, insbesondere sprungartig, ausgeführt werden, so dass die erstellten Bilder in einer Ausführung während großer Zeiträume "umgebungsfest" sind.

Durch die, insbesondere wahlweise, Vorgabe einer Geschwindigkeit, die höchstens die Hälfte der Geschwindigkeit der Relativbewegung zwischen dem Instrument und dem Aufnahmemittel beträgt, kann die Reset-Bewegung sehr langsam, insbesondere gleitend, ausgeführt werden, so dass die erstellten Bilder sich in einer Ausführung während der Reset-Bewegung relativ zu einer Umgebung nur langsam ändern.

In einer Ausführung wird die Soll-Transformation, insbesondere variabel, auf Basis einer erfassten, insbesondere aktuellen, Transformation zwischen der aufnahmemittelfesten Referenz und der instrumentenfesten Referenz vorgegeben. Insbesondere kann die Soll-Transformation eine zu einem, insbesondere manuell, bestimmten Zeitpunkt erfasste aktuelle Transformation sein. Auf diese Weise kann der Bediener in einer Ausführung eine gewünschte Perspektive der erstellten Bilder festlegen, auf die die Reset-Bewegung zurückstellt. Insbesondere kann er hierzu Instrument und Aufnahmemittel relativ zueinander in einer gewünschten Weise positionieren und eine in dieser Stellung erfasste Transformation als Soll-Transformation vorgeben.

In einer Ausführung umfasst die Soll- und aktuelle Transformation, insbesondere wahlweise, eine ein-, zwei- oder dreidimensionale, Translation und/oder eine, insbesondere ein-, zwei- oder dreidimensionale, Rotation zwischen der aufnahmemittelfesten Referenz und der instrumentenfesten Referenz insbesondere nur eine solche Translation oder nur eine solche Rotation. Insbesondere durch eine entsprechende Wahl kann in einer Ausführung die Reset-Bewegung vorteilhaft eine ein- oder mehrdimensionale Verschiebung und/oder Drehung, insbesondere um eine optische Achse des Aufnahmemittels und/oder senkrecht hierzu, aufweisen, insbesondere sein. Umfassen beispielsweise die Transformationen nur einen Abstand, insbesondere in Richtung der optischen Achse des Aufnahmemittels oder einer Bildebene senkrecht hierzu, so entspricht eine bestimmte Reset-Bewegung zur Rückführung der aktuellen Transformation auf die Soll-Transformation eine planare Verschiebung ohne Orientierungsänderung.

In einer Ausführung umfasst die Abweichung eine, insbesondere mehrdimensionale, Differenz zwischen der Soll- und der aktuellen Transformation und/oder eine, insbesondere eindimensionale, mathematische Norm, insbesondere eine Maximums- oder Betragsnorm, dieser Differenz. So kann in einer Ausführung die Abweichung der Betrag einer Differenz zwischen den Verbindungsvektoren (***x***(t₀)-***y***(t₀)) und (***x***(t)-***y***(t)) (wobei die (***x***(t₀)-***y***(t₀)) die Soll- und (***x***(t)-***y***(t)) die aktuelle Transformation ist), die größte Komponente einer, insbesondere gewichteten, Differenz von DENAVIT-HARTEN BERG-Matrizen, Euler- oder Kardanwinkeln, Quaternionen oder dergleichen, die die Soll- bzw. aktuelle Transformation beschreiben, oder dergleichen umfassen, insbesondere sein. Entsprechend kann die vorgegebene Bedingung erfüllt sein, wenn ein Abstand und/oder eine Verdrehung zwischen aufnahmemittel- und instrumentenfester Referenz einen vorgegebenen Wert übersteigt.

In einer Ausführung wird eine Position und/oder Orientierung der aufnahmemittelfesten Referenz und/oder der instrumentenfesten Referenz und/oder die Soll- und/oder aktuelle Transformation auf Basis einer erfassten Pose der Manipulatoranordnung, insbesondere von erfassten Gelenkkoordinaten, insbesondere -winkeln, eines oder mehrerer Manipulatoren der Manipulatoranordnung, ermittelt.

Aus einer erfassten Pose der das Instrument und/oder das Aufnahmemittel führenden Manipulatoranordnung kann vorteilhaft in an sich als sogenannte Vorwärtskinematik bekannter Weise eine Position und/oder Orientierung des Aufnahmemittels und des Instrumentes und damit einer aufnahmemittel(bauteil)festen Referenz und/oder einer instrumenten(bauteil)festen Referenz und/oder eine relative Position und/oder Orientierung von Aufnahmemittel und Instrument zueinander und damit eine Soll- und/oder aktuelle Transformation ermittelt werden.

Aus der auf Basis der erfassten Pose der Manipulatoranordnung ermittelten relativen Position und/oder Orientierung von Aufnahmemittel und Instrument zueinander kann in einer Ausführung auch eine relative Position und/oder Orientierung eines Abbildes des Instruments in einem von dem Aufnahmemittel erstellten Bild ermittelt und so wiederum eine relative Position und/oder Orientierung von aufnahmemittel- und instrumentenfester Referenz zueinander und damit eine Soll- und/oder aktuelle Transformation ermittelt werden. In einer Weiterbildung wird hierzu das Aufnahmemittel vorab kalibriert, insbesondere, um Parameter des Aufnahmemittels zu bestimmen.

Gleichermaßen kann eine solche relative Position und/oder Orientierung eines Abbildes des Instruments in einem von dem Aufnahmemittel erstellten Bild in einer anderen Ausführung auch mittels einer Bilderkennung, mit der das Abbild innerhalb des Bildes gesucht bzw. identifiziert wird, ermittelt und so wiederum eine relative Position und/oder Orientierung von aufnahmemittel- und instrumentenfester Referenz zueinander und damit eine Soll- und/oder aktuelle Transformation ermittelt werden.

In einer Ausführung ist die Bedingung erfüllt, sofern ein Abbild des Instruments in dem durch das Aufnahmemittel erstellten Bild ein, insbesondere variabel, vorgegebenes Kriterium erfüllt, insbesondere ein vorgegebener Bereich des Abbildes des Instruments außerhalb eines vorgegebenen Bildbereichs ist.

Zusätzlich oder alternativ ist die Bedingung in einer Ausführung erfüllt, sofern die Abweichung einen vorgegebenen ein- oder mehrdimensionalen Grenzwert übersteigt, sich also insbesondere eine Relativposition und/oder -orientierung zwischen Aufnahmemittel und Instrument, die durch eine aktuelle Transformation zwischen aufnahmemittel- und instrumentenfester Referenz beschrieben wird, gegenüber einer Ausgangsstellung, die durch die Soll-Transformation beschrieben wird, übermäßig verändert (hat). Wie vorstehend erläutert, kann dieser Grenzwert in einer Weiterbildung derart vorgegeben sein, dass die Bedingung erfüllt ist, sofern ein Abbild des Instruments in dem durch das Aufnahmemittel erstellten Bild ein, insbesondere variabel, vorgegebenes Kriterium erfüllt, insbesondere ein vorgegebener Bereich des Abbildes des Instruments außerhalb eines vorgegebenen Bildbereichs ist.

Erfindungsgemäß ist die Bedingung variabel vorgebbar. Hierdurch kann der Bediener vorteilhaft verändern, ab wann eine Reset-Bewegung vorgegeben wird.

In einer Ausführung wird die Reset-Bewegung der Manipulatoranordnung zur Rückführung der aktuellen Transformation auf die Soll-Transformation derart bestimmt, dass sich eine Position und/oder Orientierung des Instruments und/oder eine Pose eines das Instrument führenden Manipulators durch die Reset-Bewegung, wenigstens im Wesentlichen, nicht ändert. Hierdurch folgt das Aufnahmemittel dem Instrument, ohne dessen Bewegung durch den Bediener zu beeinträchtigen. Insbesondere wird in einer Ausführung nur eine Reset-Bewegung eines oder mehrerer das Aufnahmemittel führender Manipulatoren der Manipulatoranordnung bestimmt.

Nach einem Aspekt der vorliegenden Erfindung ist das Steuermittel des medizinischen Systems zur Durchführung eines hier beschriebenen Verfahrens eingerichtet oder führt ein hier beschriebenes Verfahren aus. Nach einem weiteren Aspekt der vorliegenden Erfindung weist ein Computerprogrammprodukt einen Programmcode zur Durchführung eines hier beschriebenen Verfahrens auf, der auf einem von einem Computer lesbaren Medium gespeichert ist

Ein Mittel im Sinne der vorliegenden Erfindung, insbesondere das Steuermittel des medizinischen Systems, kann hard- und/oder softwaretechnisch ausgebildet sein, insbesondere eine, vorzugsweise mit einem Speicher- und/oder Bussystem daten- bzw. signalverbundene, insbesondere digitale, Verarbeitungs-, insbesondere Mikroprozessoreinheit (CPU) und/oder ein oder mehrere Programme oder Programmmodule aufweisen. Die CPU kann dazu ausgebildet sein, Befehle, die als ein in einem Speichersystem abgelegtes Programm implementiert sind, abzuarbeiten, Eingangssignale von einem Datenbus zu erfassen und/oder Ausgangssignale an einen Datenbus abzugeben. Ein Speichersystem kann ein oder mehrere, insbesondere verschiedene, Speichermedien, insbesondere optische, magnetische, Festkörper- und/oder andere nicht-flüchtige Medien aufweisen. Das Programm kann derart beschaffen sein, dass es die hier beschriebenen Verfahren verkörpert bzw. auszuführen imstande ist, sodass die CPU die Schritte solcher Verfahren ausführen kann und damit insbesondere die Soll-Bewegung vorgeben kann.

Weitere Vorteile und Merkmale ergeben sich aus den Unteransprüchen und den Ausführungsbeispielen. Hierzu zeigt, teilweise schematisiert:
- Fig. 1:: einen Teil eines medizinischen Systems mit einer Manipulatoranordnung nach einer Ausführung der vorliegenden Erfindung; und
- Fig. 2:: ein Verfahren zum automatischen Vorgeben einer Soll-Bewegung der Manipulatoranordnung.

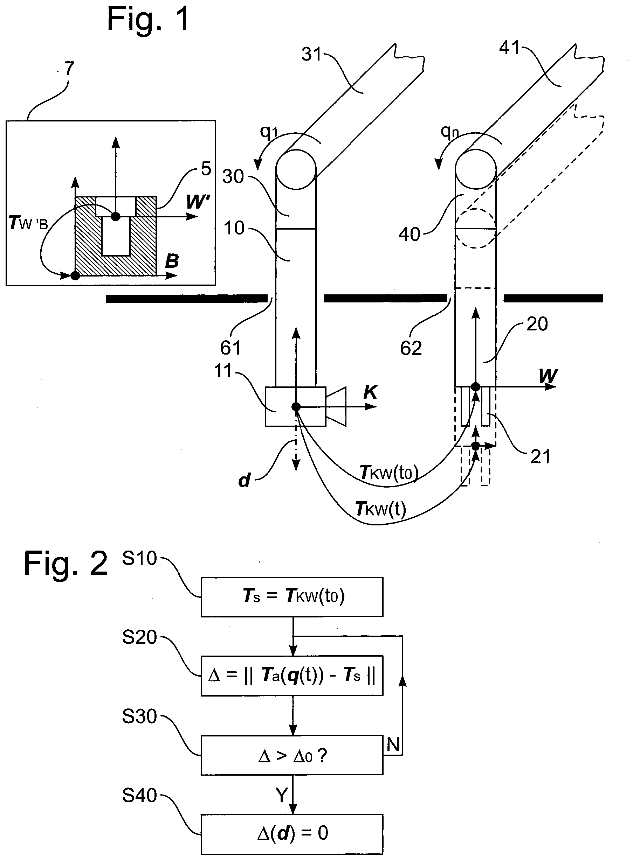

Fig. 1 zeigt einen Teil eines medizinischen Systems nach einer Ausführung der vorliegenden Erfindung.

Das medizinische System weist eine Manipulatoranordnung mit zwei Manipulatoren auf, von denen in Fig. 1 nur jeweils ein Endflansch 30 bzw. 40 und ein Teil eines mit diesem über ein Drehgelenk mit Drehwinkel q₁ bzw. qₙ verbundenen Arms 31 bzw. 41 angedeutet ist.

Das medizinische System weist weiter ein zum minimalinvasiven Einsatz ausgebildetes medizinisches Instrument mit einem Instrumentenschaft 20 auf, der durch eine Körperöffnung 62 in einen Patienten eingeführt ist, und einen relativ zu dem Instrumentenschaft aktuiert bewegbaren Endeffektor 21 zum Klemmen oder dergleichen auf. Der Instrumentenschaft 20 ist lösbar mit dem Endflansch 40 verbunden und so mittels der Manipulatoranordnung bewegbar bzw. durch diese geführt.

Das medizinische System weist weiter ein zum minimalinvasiven Einsatz ausgebildetes Aufnahmemittel zum Erstellen von Bildern mit einem Schaft 10 auf, der durch eine Körperöffnung 61 in den Patienten eingeführt ist, und einen relativ zu dem Schaft ortsfesten Endeffektor 11 in Form einer Mikrokamera auf, die sichtbare elektromagnetische Strahlung intrakorporal empfängt, die das Aufnahmemittel weiterverarbeitet, um Bilder 5 von dem Endeffektor 21 und dessen Operationsumgebung zu erstellen. Der Schaft 10 ist lösbar mit dem Endflansch 30 verbunden und so mittels der Manipulatoranordnung bewegbar bzw. durch diese geführt.

Das medizinische System weist weiter ein Steuermittel 7 in Form eines Rechners auf. Dieses führt ein Verfahren durch, welches nachfolgend mit Bezug auf Fig. 2 erläutert wird:
Zunächst wird in einem Schritt S10 eine Soll-Transformation ***T***ₛ = ***T***_{KW}(t₀) zwischen einer aufnahmemittelfesten Referenz und einer instrumentenfesten Referenz ermittelt.

Die aufnahmemittelfeste Referenz kann exemplarisch, wie in Fig. 1 angedeutet, ein Koordinatensystem ***K*** sein, welches ortsfest bezüglich der Kamera 11 ist. Gleichermaßen kann die aufnahmemittelfeste Referenz zum Beispiel auch ein Koordinatensystem ***B*** sein, welches ortsfest bezüglich des von der Kamera erstellten Bildes 5 ist.

In analoger Weise kann die instrumentenfeste Referenz exemplarisch, wie in Fig. 1 angedeutet, ein Koordinatensystem ***W*** sein, welches ortsfest bezüglich des Endeffektors 21 oder Instrumentenschaftes 20 ist. Gleichermaßen kann die instrumentenfeste Referenz zum Beispiel auch ein Koordinatensystem ***W***' sein, welches ortsfest bezüglich des Abbildes des Instruments in dem von der Kamera erstellten Bildes 5 ist.

Die Transformation ist in Fig. 1 exemplarisch durch die Abbildung ***T***_{KW} zwischen den Koordinatensystemen ***K*** und ***W*** bzw. die Abbildung ***T***_{W'B} zwischen den Koordinatensystemen ***W***' und ***B*** angedeutet. Sie beschreibt die relative Position und Orientierung der Kamera 11 und des Instrumentenschafts 20 bzw. -endeffektors 21 bzw. des erstellen Bildes 5 und des Instrumentenabbildes darin.

Die Transformation ***T***_{KW} wird jeweils auf Basis der erfassten Gelenkwinkel ***q*** = (q₁,...,qₙ) bzw. einer erfassten Pose der Manipulatoranordnung mittels Vorwärtskinematik ermittelt.

Die Transformation ***T***_{W'B} kann unter Berücksichtigung eines Blickfeldes der Kamera 11 ebenfalls jeweils auf Basis der erfassten Gelenkwinkel ***q*** ermittelt werden. Gleichermaßen kann die Transformation ***T***_{W'B}, die eine relative Position und/oder Orientierung des Abbildes des Endeffektors 21 des Instruments in dem von dem Aufnahmemittel 11 erstellten Bild 5 vermittelt, auch mittels einer Bilderkennung ermittelt werden.

Die Soll-Transformation wird in Schritt S10 auf Basis einer zum Zeitpunkt t₀ erfassten aktuellen Transformation zwischen der aufnahmemittelfesten Referenz und der instrumentenfesten Referenz vorgegeben, im Ausführungsbeispiel der Fig. 2 exemplarisch durch ***T***ₛ = ***T***_{KW}(t₀) angedeutet.

Dann wird periodisch oder kontinuierlich in Schritt S20 eine durch "∥...∥" angedeutete mathematische Norm einer Differenz zwischen der Soll- und einer fortlaufend erfassten aktuellen Transformation ***T***ₐ(***q***(t)), die aus den Gelenkwinkeln ***q*** = (q₁,...,qₙ) der Manipulatoranordnung ermittelt wird, als eine Abweichung Δ zwischen der Soll-Transformation und der jeweils aktuellen Transformation zwischen der aufnahmemittelfesten Referenz und der instrumentenfesten Referenz ermittelt und in Schritt S30 überwacht, ob diese Abweichung Δ einen vorgegebenen Grenzwert Δ₀ übersteigt, sich also insbesondere eine Relativposition und/oder -orientierung zwischen Aufnahmemittel und Instrument übermäßig verändert hat.

Sobald die Abweichung Δ den vorgegebenen Grenzwert Δ₀ übersteigt (S30: "Y"), was in Fig. 1 exemplarisch durch eine gestrichelt dargestellte ausgelenkte Stellung des medizinischen Instruments angedeutet ist, ist damit eine vorgegebene Bedingung erfüllt und das Steuermittel 7 bestimmt in einem Schritt S40 eine Reset-Bewegung ***d*** der Manipulatoranordnung zur Rückführung der aktuellen Transformation auf die Soll-Transformation, die in Fig. 1 zur Illustration strichpunktiert angedeutet ist.

Im Ausführungsbeispiel ist diese Bedingung derart vorgegeben, dass sie erfüllt ist, sofern das Abbild des Instruments in dem durch das Aufnahmemittel erstellten Bild 5 außerhalb eines vorgegebenen Bildbereichs ist.

Die Reset-Bewegung ***d*** wird in Schritt S40 auf Basis der Abweichung Δ bei Erfüllung der Bedingung (S30: "Y") derart bestimmt, dass diese Abweichung sich, vorzugsweise auf Null, reduziert (Δ(***d***) = 0), beispielsweise durch Lösen eines Minimierungsproblems "***d*** so, dass Δ(***d***) = Minimum!" oder dergleichen.

Anschließend kann das Steuermittel diese vorgegebene Soll- bzw. Reset-Bewegung durch entsprechende Aktuierung der Gelenke der Manipulatoranordnung umsetzen (in Fig. 2 nicht dargestellt).

Dabei kann die Reset-Bewegung im Ausführungsbeispiel wahlweise mit einer Geschwindigkeit dd/dt vorgegeben werden, die wenigstens das Doppelte oder höchstens die Hälfte einer Geschwindigkeit einer Relativbewegung zwischen dem Instrument und dem Aufnahmemittel beträgt.

Die Reset-Bewegung der Manipulatoranordnung zur Rückführung der aktuellen Transformation auf die Soll-Transformation wird in Schritt S40 derart bestimmt, dass sich eine Position und/oder Orientierung des Endeffektors 21 des Instruments dadurch nicht ändert. Entsprechend wird das oben genannte Minimierungsproblem nur unter Variation der Gelenkwinkel des Manipulators 30, 31 gelöst, der die Kamera 11 führt.

Obwohl in der vorhergehenden Beschreibung exemplarische Ausführungen erläutert wurden, sei darauf hingewiesen, dass eine Vielzahl von Abwandlungen möglich ist. Außerdem sei darauf hingewiesen, dass es sich bei den exemplarischen Ausführungen lediglich um Beispiele handelt, die den Schutzbereich, die Anwendungen und den Aufbau in keiner Weise einschränken sollen. Vielmehr wird dem Fachmann durch die vorausgehende Beschreibung ein Leitfaden für die Umsetzung von mindestens einer exemplarischen Ausführung gegeben, wobei diverse Änderungen, insbesondere in Hinblick auf die Funktion und Anordnung der beschriebenen Bestandteile, vorgenommen Werden können, ohne den Schutzbereich zu verlassen, wie er sich aus den Ansprüchen und diesen äquivalenten Merkmalskombinationen ergibt.

### Bezuqszeichenliste

- 10: Schaft (Aufnahmemittel)
- 11: Kamera (Aufnahmemittel)
- 20: Instrumentenschaft
- 21: Endeffektor
- 30, 40: Endflansch (Manipulator)
- 31, 41: Arm (Manipulator)
- 5: Bild
- 61, 62: Körperöffnung
- 7: Steuermittel

- ***d***: Reset-Bewegung
- ***B***: aufnahmemittelbildfeste Referenz
- ***K***: aufnahmemittelbauteilfeste Referenz
- **q**₁, **q**ₙ, ***q***: Gelenkwinkel
- ***T***: Transformation
- ***W***: instrumentenbauteilfeste Referenz
- ***W**'*: instrumentenabbildfeste Referenz
- Δ: Abweichung

## Patentansprüche

1. Medizinisches System mit einer Manipulatoranordnung (30, 31, 40, 41), einem medizinischen Instrument (20, 21), einem Aufnahmemittel (10, 11) zum Erstellen von Bildern (5) und einem Steuermittel (7), wobei das Aufnahmemittel durch die Manipulatoranordnung geführt ist und das Steuermittel zur Durchführung eines Verfahrens zum automatischen Vorgeben einer Soll-Bewegung (***d***) der Manipulatoranordnung (30, 31, 40, 41) eingerichtet ist, wobei das Verfahren, zu dessen Durchführung das Steuermittel eingerichtet ist, die Schritte aufweist:
Ermitteln (S10) einer Soll-Transformation (***T***ₛ) zwischen einer aufnahmemittelfesten Referenz (***K***, ***B***) und einer instrumentenfesten Referenz (***W***, ***W*'**);
Überwachen (S20, S30) einer Abweichung (Δ) zwischen der Soll-Transformation und einer aktuellen Transformation (***T***_{KW}(t)) zwischen der aufnahmemittelfesten Referenz und der instrumentenfesten Referenz; und
Bestimmen (S40) einer Reset- Bewegung (***d***) der Manipulatoranordnung zur Rückführung der aktuellen Transformation auf die Soll-Transformation, wenn die Abweichung eine vorgegebene Bedingung (Δ>Δ₀) erfüllt, **dadurch gekennzeichnet dass** dadurch, dass die
Bedingung variabel vorgebbar ist, ein Bediener verändern kann, ab wann eine Reset-Bewegung vorgegeben wird.

2. Medizinisches System nach dem vorhergehenden Anspruch, **dadurch gekennzeichnet, dass** das Steuermittel derart eingerichtet ist, dass bei dem Verfahren, zu dessen Durchführung das Steuermittel eingerichtet ist, die Reset-Bewegung auf Basis der Abweichung, insbesondere bei Erfüllung der Bedingung, und/oder unabhängig von einer Relativbewegung zwischen dem Instrument und dem Aufnahmemittel nach Erfüllung der Bedingung vorgegeben wird; und/oder **dadurch gekennzeichnet, dass** bei dem Verfahren, zu dessen Durchführung das Steuermittel eingerichtet ist, die Reset-Bewegung mit einer, insbesondere maximalen oder konstanten, Geschwindigkeit vorgegeben wird, die wenigstens das Doppelte oder höchstens die Hälfte einer Geschwindigkeit einer Relativbewegung zwischen dem Instrument und dem Aufnahmemittel beträgt.

3. Medizinisches System nach einem der vorhergehenden Ansprüche, **dadurch gekennzeichnet, dass** bei dem Verfahren, zu dessen Durchführung das Steuermittel eingerichtet ist, die Soll-Transformation, insbesondere variabel, auf Basis einer erfassten Transformation (***T***_{KW}(t₀)) zwischen der aufnahmemittelfesten Referenz und der instrumentenfesten Referenz vorgegeben wird; und/oder **dadurch gekennzeichnet, dass** bei dem Verfahren, zu dessen Durchführung das Steuermittel eingerichtet ist, die Soll- und aktuelle Transformation eine, insbesondere ein-, zwei- oder dreidimensionale, Translation und/oder eine, insbesondere ein-, zwei- oder dreidimensionale, Rotation zwischen der aufnahmemittelfesten Referenz und der instrumentenfesten Referenz umfasst.

4. Medizinisches System nach einem der vorhergehenden Ansprüche, **dadurch gekennzeichnet, dass** bei dem Verfahren, zu dessen Durchführung das Steuermittel eingerichtet ist, die Abweichung eine, insbesondere eindimensionale, Norm und/oder eine, insbesondere mehrdimensionale, Differenz umfasst.

5. Medizinisches System nach einem der vorhergehenden Ansprüche, **dadurch gekennzeichnet, dass** bei dem Verfahren, zu dessen Durchführung das Steuermittel eingerichtet ist, die Bedingung erfüllt ist, sofern ein durch das Aufnahmemittel erstelltes Bild des Instruments ein, insbesondere variabel, vorgegebenes Kriterium erfüllt.

6. Medizinisches System nach einem der vorhergehenden Ansprüche, **dadurch gekennzeichnet, dass** das Aufnahmemittel zum, insbesondere intra- und/oder extrakorporalen, Aussenden und/oder Empfangen von, insbesondere sichtbarer oder unsichtbarer, elektromagnetischer Strahlung und/oder Ultraschall ausgebildet ist.

7. Medizinisches System nach einem der vorhergehenden Ansprüche, **dadurch gekennzeichnet, dass** das Aufnahmemittel und/oder Instrument zum minimalinvasiven Einsatz ausgebildet sind und/oder einen mittels der Manipulatoranordnung bewegbaren Schaft (10, 20) zum Einführen durch eine Körperöffnung (61, 62) und einen Endeffektor (11, 21) aufweisen und/oder dass auch das Instrument durch die Manipulatoranordnung geführt ist.

8. Computerprogrammprodukt mit einem Programmcode, der auf einem von einem Computer lesbaren Medium gespeichert ist und bei der Ausführung durch ein Steuermittel (7) eines medizinischen Systems, insbesondere nach einem der vorhergehenden Ansprüche, das Steuermittel veranlasst, ein Verfahren zum automatischen Vorgeben einer Soll-Bewegung (***d***) einer Manipulatoranordnung (30, 31, 40, 41) des medizinischen Systems durchzuführen, wobei das medizinische System ein medizinisches Instrument (20, 21) und ein Aufnahmemittel (10, 11) zum Erstellen von Bildern (5) aufweist, wobei das Aufnahmemittel durch die Manipulatoranordnung geführt ist, und wobei das Verfahren die Schritte aufweist:
Ermitteln (S10) einer Soll-Transformation (***T***ₛ) zwischen einer aufnahmemittelfesten Referenz (***K***, ***B***) und einer instrumentenfesten Referenz (***W***, ***W'***);
Überwachen (S20, S30) einer Abweichung (Δ) zwischen der Soll-Transformation und einer aktuellen Transformation (***T***_{KW}(t)) zwischen der aufnahmemittelfesten Referenz und der instrumentenfesten Referenz; und
Bestimmen (S40) einer Reset- Bewegung (***d***) der Manipulatoranordnung zur Rückführung der aktuellen Transformation auf die Soll-Transformation, wenn die Abweichung eine vorgegebene Bedingung (Δ>Δ₀) erfüllt, **dadurch gekennzeichnet dass** dadurch, dass die
Bedingung variabel vorgebbar ist, ein Bediener verändern kann, ab wann eine Reset-Bewegung vorgegeben wird.

9. Computerprogrammprodukt nach dem vorhergehenden Anspruch, **dadurch gekennzeichnet, dass** bei dem Verfahren die Reset-Bewegung auf Basis der Abweichung, insbesondere bei Erfüllung der Bedingung, und/oder unabhängig von einer Relativbewegung zwischen dem Instrument und dem Aufnahmemittel nach Erfüllung der Bedingung vorgegeben wird; und/oder **dadurch gekennzeichnet, dass** bei dem Verfahren die Reset-Bewegung mit einer, insbesondere maximalen oder konstanten, Geschwindigkeit vorgegeben wird, die wenigstens das Doppelte oder höchstens die Hälfte einer Geschwindigkeit einer Relativbewegung zwischen dem Instrument und dem Aufnahmemittel beträgt.

10. Computerprogrammprodukt nachAnspruch 8 oder 9, **dadurch gekennzeichnet, dass** bei dem Verfahren die Soll-Transformation, insbesondere variabel, auf Basis einer erfassten Transformation (***T***_{KW}(t₀)) zwischen der aufnahmemittelfesten Referenz und der instrumentenfesten Referenz vorgegeben wird; und/oder **dadurch gekennzeichnet, dass** bei dem Verfahren die Soll- und aktuelle Transformation eine, insbesondere ein-, zwei- oder dreidimensionale, Translation und/oder eine, insbesondere ein-, zwei- oder dreidimensionale, Rotation zwischen der aufnahmemittelfesten Referenz und der instrumentenfesten Referenz umfasst.

11. Computerprogrammprodukt nach einem der Ansprüche 8 bis 10, **dadurch gekennzeichnet, dass** bei dem Verfahren die Abweichung eine, insbesondere eindimensionale, Norm und/oder eine, insbesondere mehrdimensionale, Differenz umfasst.

12. Computerprogrammprodukt nach einem der Ansprüche 8 bis 11, **dadurch gekennzeichnet, dass** bei dem Verfahren die Bedingung erfüllt ist, sofern ein durch das Aufnahmemittel erstelltes Bild des Instruments ein, insbesondere variabel, vorgegebenes Kriterium erfüllt.

13. Computerprogrammprodukt nach einem der Ansprüche 8 bis 12, **dadurch gekennzeichnet, dass** das Aufnahmemittel und/oder Instrument zum minimalinvasiven Einsatz ausgebildet sind und/oder einen mittels der Manipulatoranordnung bewegbaren Schaft (10, 20) zum Einführen durch eine Körperöffnung (61, 62) und einen Endeffektor (11, 21) aufweisen und/oder dass auch das Instrument durch die Manipulatoranordnung geführt ist.

## Claims

1. A medical system which comprises a manipulator arrangement (30, 31, 40, 41), a medical instrument (20, 21), an image acquisition means (10, 11) for acquiring images (5) and a control means (7), wherein the image acquisition means is guided by the manipulator arrangement and the control means is arranged to carry out a method of automatically specifying a target movement (d) of the manipulator arrangement (30, 31, 40, 41), wherein the method which the control means is arranged to carry out comprises the steps of:
determining (S10) a target transformation (***T***ₛ) between a reference (***K***, ***B***) which is fixed with respect to the image acquisition means and a reference (***W***, ***W'***) which is fixed with respect to the instrument;
monitoring (S20, S30) a deviation (Δ) between the target transformation and a current transformation (***T***_{KW} (t)) between the reference which is fixed with respect to the image acquisition means and the reference which is fixed with respect to the instrument; and
determining (S40) a reset movement (***d***) of the manipulator arrangement for returning the current transformation to the target transformation if the deviation satisfies a specified condition (Δ > Δ₀), **characterised in that** an operator can change from when a reset movement is prescribed, the reason for this being that the condition can be specified in a variable manner.

2. The medical system according to the preceding claim, **characterised in that** the control means is arranged in such a way that, in the method which the control means is arranged to carry out, the reset movement is specified on the basis of the deviation, in particular upon the condition having been satisfied, and / or independently of a relative movement between the instrument and the image acquisition means after the condition has been satisfied; and / or **characterised in that**, in the method which the control means is arranged to carry out, the reset movement is specified at a speed, in particular a maximum or constant speed, which is at least twice or at most half a speed of a relative movement between the instrument and the image acquisition means.

3. The medical system according to any one of the preceding claims, **characterised in that**, in the method which the control means is arranged to carry out, the target transformation is specified, in particular in a variable manner, on the basis of a detected transformation (***T***_{KW} (t₀)) between the reference which is fixed with respect to the image acquisition means and the reference which is fixed with respect to the instrument; and / or **characterised in that**, in the method which the control means is arranged to carry out, the target transformation and the current transformation comprise a translational movement, in particular a one-dimensional, two-dimensional or three-dimensional translational movement, and / or a rotational movement, in particular a one-dimensional, two-dimensional or three-dimensional rotational movement between the reference which is fixed with respect to the image acquisition means and the reference which is fixed with respect to the instrument.

4. The medical system according to any one of the preceding claims, **characterised in that**, in the method which the control means is arranged to carry out, the deviation comprises a norm, in particular a one-dimensional norm, and / or a difference, in particular a multi-dimensional difference.

5. The medical system according to any one of the preceding claims, **characterised in that**, in the method which the control means is arranged to carry out, the condition is satisfied if an image of the instrument, which image has been generated by the image acquisition means, satisfies a specified criterion, in particular a criterion which has been specified in a variable manner.

6. The medical system according to any one of the preceding claims, **characterised in that** the image acquisition means is constructed for the transmission and / or reception of electromagnetic radiation, in particular visible or invisible electromagnetic radiation, and / or ultrasound, in particular for the intracorporeal and / or extracorporeal transmission and / or reception of electromagnetic radiation, in particular visible or invisible electromagnetic radiation, and / or ultrasound.

7. The medical system according to any one of the preceding claims, **characterised in that** the image acquisition means and / or the instrument are constructed for minimally invasive use and / or **in that** they comprise a shaft (10, 20) which can be moved by means of the manipulator arrangement, the shaft (10, 20) being for insertion through a body opening (61, 62), and an end effector (11, 21), and / or **in that** the instrument is also guided by the manipulator arrangement.

8. A computer program product which comprises a program code which is stored on a computer-readable medium and which, when it is being executed by a control means (7) of a medical system, in particular a medical system according to any one of the preceding claims, causes the control means to carry out a method of automatically specifying a target movement (d) of a manipulator arrangement (30, 31, 40, 41) of the medical system, wherein the medical system comprises a medical instrument (20, 21) and an image acquisition means (10, 11) for acquiring images (5), wherein the image acquisition means is guided by the manipulator arrangement, and wherein the method comprises the steps of:
determining (S10) a target transformation (***T***ₛ) between a reference (***K***, ***B***) which is fixed with respect to the image acquisition means and a reference (***W***, ***W'***) which is fixed with respect to the instrument;
monitoring (S20, S30) a deviation (Δ) between the target transformation and a current transformation (***T***_{KW} (t)) between the reference which is fixed with respect to the image acquisition means and the reference which is fixed with respect to the instrument; and
determining (S40) a reset movement (***d***) of the manipulator arrangement for returning the current transformation to the target transformation if the deviation satisfies a specified condition (Δ > Δ₀), **characterised in that** an operator can change from when a reset movement is prescribed, the reason for this being that the condition can be specified in a variable manner.

9. The computer program product according to the preceding claim, **characterised in that**, in the method, the reset movement is specified on the basis of the deviation, in particular upon the condition having been satisfied, and / or independently of a relative movement between the instrument and the image acquisition means after the condition has been satisfied; and / or **characterised in that**, in the method, the reset movement is specified at a speed, in particular a maximum or constant speed, which is at least twice or at most half a speed of a relative movement between the instrument and the image acquisition means.

10. The computer program product according to claim 8 or 9, **characterised in that**, in the method, the target transformation is specified, in particular in a variable manner, on the basis of a detected transformation (***T***_{KW} (t₀)) between the reference which is fixed with respect to the image acquisition means and the reference which is fixed with respect to the instrument; and / or **characterised in that**, in the method, the target transformation and the current transformation comprise a translational movement, in particular a one-dimensional, two-dimensional or three-dimensional translational movement, and / or a rotational movement, in particular a one-dimensional, two-dimensional or three-dimensional rotational movement between the reference which is fixed with respect to the image acquisition means and the reference which is fixed with respect to the instrument.

11. The computer program product according to any one of the claims 8 to 10, **characterised in that**, in the method, the deviation comprises a norm, in particular a one-dimensional norm, and / or a difference, in particular a multi-dimensional difference.

12. The computer program product according to any one of the claims 8 to 11, **characterised in that**, in the method, the condition is satisfied if an image of the instrument, which image has been generated by the image acquisition means, satisfies a specified criterion, in particular a criterion which has been specified in a variable manner

13. The computer program product according to any one of the claims 8 to 12, **characterised in that** the image acquisition means and / or the instrument are constructed for minimally invasive use and / or **in that** they comprise a shaft (10, 20) which can be moved by means of the manipulator arrangement, the shaft (10, 20) being for insertion through a body opening (61, 62), and an end effector (11, 21), and / or **in that** the instrument is also guided by the manipulator arrangement.

## Revendications

1. Système médical avec un ensemble de manipulation (30, 31, 40, 41), un instrument médical (20, 21), un moyen d'enregistrement (10, 11) destiné à créer des images (5) et un moyen de commande (7), dans lequel le moyen d'enregistrement est guidé par l'ensemble de manipulation et le moyen de commande est mis au point pour mettre en œuvre un procédé de spécification automatique d'un déplacement de consigne (d) de l'ensemble de manipulation (30, 31, 40, 41), dans lequel le procédé, pour la mise en œuvre duquel le moyen de commande est mis au point, présente les étapes :
de détermination (S10) d'une transformation de consigne (Ts) entre une référence spécifique au moyen d'enregistrement (K, B) et une référence spécifique à l'instrument (W, W') ;
de surveillance (S20, S30) d'un écart (Δ) entre la transformation de consigne et une transformation instantanée (T_{KW(t)}) entre la référence spécifique au moyen d'enregistrement et la référence spécifique à l'instrument ; et
de définition (S40) d'un déplacement de réinitialisation (d) de l'ensemble de manipulation pour ramener la transformation instantanée à la transformation de consigne lorsque l'écart satisfait à une condition spécifiée (Δ > Δ₀), **caractérisé en ce que** du fait que la condition peut être spécifiée de manière variable, un utilisateur peut modifier le moment à partir duquel un déplacement de réinitialisation est spécifié.

2. Système médical selon la revendication précédente, **caractérisé en ce que** le moyen de commande est mis au point de telle manière que lors du procédé, pour la mise en œuvre duquel le moyen de commande est mis au point, le déplacement de réinitialisation est spécifié sur la base de l'écart, en particulier lors de la satisfaction de la condition, et/ou indépendamment d'un déplacement relatif entre l'instrument et le moyen d'enregistrement après satisfaction de la condition ; et/ou
**caractérisé en ce que** lors du procédé, pour la mise en œuvre duquel le moyen de commande est mis au point, le déplacement de réinitialisation est spécifié avec une vitesse en particulier maximale ou constante, qui est égale au moins au double ou au maximum à la moitié d'une vitesse d'un déplacement relatif entre l'instrument et le moyen d'enregistrement.

3. Système médical selon l'une quelconque des revendications précédentes, **caractérisé en ce que** lors du procédé, pour la mise en œuvre duquel le moyen de commande est mis au point, la transformation de consigne est spécifiée en particulier de manière variable sur la base d'une transformation détectée (T_{KW(t0)}) entre la référence spécifique au moyen d'enregistrement et la référence spécifique à l'instrument ; et/ou
**caractérisé en ce que** lors du procédé, pour la mise en œuvre duquel le moyen de commande est mis au point, la transformation de consigne et la transformation instantanée comprennent une translation en particulier uni-, bi- ou tridimensionnelle et/ou une rotation en particulier uni-, bi- ou tridimensionnelle entre la référence spécifique au moyen d'enregistrement et la référence spécifique à l'instrument.

4. Système médical selon l'une quelconque des revendications précédentes, **caractérisé en ce que** lors du procédé, pour la mise en œuvre duquel le moyen de commande est mis au point, l'écart comprend une norme en particulier unidimensionnelle et/ou une différence en particulier multidimensionnelle.

5. Système médical selon l'une quelconque des revendications précédentes, **caractérisé en ce que** lors du procédé, pour la mise en œuvre duquel le moyen de commande est mis au point, la condition est satisfaite, dans la mesure où une image de l'instrument créée par le moyen d'enregistrement satisfait à un critère spécifié en particulier de manière variable.

6. Système médical selon l'une quelconque des revendications précédentes, **caractérisé en ce que** le moyen d'enregistrement est réalisé pour envoyer et/ou recevoir en particulier de manière intra- et/ou extracorporelle un rayonnement électromagnétique en particulier visible ou invisible et/ou des ultrasons.

7. Système médical selon l'une quelconque des revendications précédentes, **caractérisé en ce que** le moyen d'enregistrement et/ou l'instrument sont réalisés pour un emploi avec une technique invasive minimale et/ou présentent une tige (10, 20) pouvant être déplacée au moyen de l'ensemble de manipulation, destinée à être introduite à travers un orifice corporel (61, 62) et un effecteur terminal (11, 21), et/ou que l'instrument est également guidé par l'ensemble de manipulation.

8. Produit-programme d'ordinateur avec un code de programme, qui est mémorisé sur un support lisible par un ordinateur et qui déclenche lors de l'exécution par un moyen de commande (7) d'un système médical, en particulier selon l'une quelconque des revendications précédentes, la mise en œuvre par le moyen de commande d'un procédé de spécification automatique d'un déplacement de consigne (d) d'un ensemble de manipulation (30, 31, 40, 41) du système médical, dans lequel le système médical présente un instrument médical (20, 21) et un moyen d'enregistrement (10, 11) pour créer des images (5), dans lequel le moyen d'enregistrement est guidé par l'ensemble de manipulation, et dans lequel le procédé présente les étapes :
de détermination (S10) d'une transformation de consigne (Ts) entre une référence spécifique au moyen d'enregistrement (K, B) et une référence spécifique à l'instrument (W, W') ;
de surveillance (S20, S30) d'un écart (Δ) entre la transformation de consigne et une transformation instantanée (T_{KW(t)}) entre la référence spécifique au moyen d'enregistrement et la référence spécifique à l'instrument ; et
de définition (S40) d'un déplacement de réinitialisation (d) de l'ensemble de manipulation pour ramener la transformation instantanée à la transformation de consigne lorsque l'écart satisfait à une condition spécifiée (Δ > Δ₀), **caractérisé en ce que** du fait que la condition peut être spécifiée de manière variable, un utilisateur peut modifier le moment à partir duquel un déplacement de réinitialisation est spécifié.

9. Produit-programme d'ordinateur selon la revendication précédente, **caractérisé en ce que** lors du procédé, le déplacement de réinitialisation est spécifié sur la base de l'écart, en particulier lors de la satisfaction de la condition et/ou indépendamment d'un déplacement relatif entre l'instrument et le moyen d'enregistrement après satisfaction de la condition ; et/ou **caractérisé en ce que** lors du procédé, le déplacement de réinitialisation est spécifié avec une vitesse en particulier maximale ou constante, qui est égale au moins au double ou au maximum à la moitié d'une vitesse d'un déplacement relatif entre l'instrument et le moyen d'enregistrement.

10. Produit-programme d'ordinateur selon la revendication 8 ou 9, **caractérisé en ce que** lors du procédé, la transformation de consigne est spécifiée en particulier de manière variable sur la base d'une transformation détectée (T_{KW(t0)}) entre la référence spécifique au moyen d'enregistrement et la référence spécifique à l'instrument ; et/ou
**caractérisé en ce que** lors du procédé la transformation de consigne et la transformation instantanée comprennent une translation en particulier uni-, bi- ou tridimensionnelle et/ou une rotation en particulier uni-, bi- ou tridimensionnelle entre la référence spécifique au moyen d'enregistrement et la référence spécifique à l'instrument.

11. Produit-programme d'ordinateur selon l'une quelconque des revendications 8 à 10, **caractérisé en ce que** lors du procédé, l'écart comprend une norme en particulier unidimensionnelle et/ou une différence en particulier multidimensionnelle.

12. Produit-programme d'ordinateur selon l'une quelconque des revendications 8 à 11, **caractérisé en ce que** lors du procédé la condition est satisfaite dans la mesure où une image de l'instrument créée par le moyen d'enregistrement satisfait à un critère spécifié en particulier de manière variable.

13. Produit-programme d'ordinateur selon l'une quelconque des revendications 8 à 12, **caractérisé en ce que** le moyen d'enregistrement et/ou l'instrument sont réalisés pour un emploi avec une technique invasive minimale et/ou présentent une tige (10, 20) pouvant être déplacée au moyen de l'ensemble de manipulation destinée à être introduite à travers un orifice corporel (61, 62) et un effecteur terminal (11, 21), et/ou que l'instrument est également guidé par l'ensemble de manipulation.
